# EUROPEAN PATENT APPLICATION

(11) **EP 3 925 565 A1**
(43) Date of publication of application: **22.12.2021**
(21) Application number: 20182587.4
(22) Date of filing: 26.06.2020
(51) Int. Cl.: A61B 50/33, A61B 50/36, A61B 50/30, A61B 50/20

(54) **APPLICATOR FOR HANDLING MEDICAL SUPPLIES AND COMPLEMENTARY TRAY FOR ORGANIZING THE PRE- AND POST-USE MEDICAL SUPPLIES**

(30) Priority: 17.06.2020 US 202016946327
(71) Applicant: Althoff, Tabitha, Oconomowoc, Wisconsin (US)
(72) Inventor: Althoff, Tabitha, Oconomowoc, Wisconsin (US)
(74) Representative: Radkov, Stoyan Atanassov

(57) **Abstract**

A disposable applicator and disposable tray for handling, drying, and separately organizing medical supplies during a medical procedure are provided. The disposable applicator can be used for drying medical supplies. The disposable tray provides two spaced apart recesses for organizing and separating pre-use and post-use medical supplies for preventing cross contamination. The post-use medical supplies, disposable applicator, and the disposable tray can be discarded after the related medical procedure to further prevent the spread of bacteria and viruses.

## Description

### BACKGROUND OF THE INVENTION

The present invention relates to medical devices and, more particularly, to a disposable applicator to assist in handling medical supplies and an operatively associated disposable tray to separately holding medical supplies throughout the medical procedure.

When handling re-usable medical supplies, such as electroencephalography (EEG) leads, cross contamination between patients is one of the biggest concerns.

Current solutions for handling re-usable medical supplies between uses are expensive, heavy, reusable, rust-prone and hard to clean thoroughly to ensure infection control standards are being met. Furthermore, these solutions do not incorporate a tray to separately accommodate the medical supplies during the different phases of the related medical procedure.

As can be seen, there is a need for a disposable applicator to assist in handling medical supplies, such as the drying of EEG leads, and a disposable tray to separately hold related supplies throughout the phases or stages of a medical procedure; thereby, preventing cross contamination between patients.

The medical device(s) embodied in the present invention are single-use, disposable device(s), eliminating the chance of cross contamination between patients. Making the items disposable enables a lighter and more affordable design. Furthermore, the disposable applicator is adapted to removably secured to the disposable tray, which will hold and keep separate pre-use and post-use medical supplies in concert with the stages of the related medical procedure. The present invention may be individually configured or bundled together. Once the use of either medical device is no longer needed, they are discarded appropriately.

### SUMMARY OF THE INVENTION

In one aspect of the present invention, disposable medical supply handling kit includes the following: a disposable applicator; a disposable tray having a storage retainer for removably securing the disposable applicator; and the disposable tray having two spaced apart recesses: a first recess and a second recess.

In another aspect of the present invention, a disposable tray for managing both pre-use and post-use medical supplies includes the following: a first recess spaced apart from a second recess; and one or more storage retainers disposed adjacent the first or second recesses.

In yet another aspect of the present invention, a method for preventing the contamination of pre-use medical supplies by post-use medical supplies includes the following: providing the above-mentioned disposable tray; placing pre-use medical supplies in the first recess; and placing post-use medical supplies in the second recess.

These and other features, aspects and advantages of the present invention will become better understood with reference to the following drawings, description and claims.

### BRIEF DESCRIPTION OF THE DRAWINGS

FIG. 1 is a perspective view of an exemplary embodiment of the present invention, shown with a disposable adapted fluidly connected to an air supply and removably secured in a disposable tray;
FIG. 2 is a perspective view of an exemplary embodiment of the present invention, shown in sterile packaging;
FIG. 3 is a partial exploded view of an exemplary embodiment of the present invention, illustrating the placement of the disposable applicator in a storage retainer;
FIG. 4 is a perspective view of an exemplary embodiment of the present invention, shown with the disposable applicator and other used supplies ready for disposal/recycling in the second recess of the disposable tray;
FIG.5 is a section view of an exemplary embodiment of the present invention, taken along line 5-5 in FIG. 3, showing only the disposable applicator;
FIG. 6 is a section view of an exemplary embodiment of the present invention, taken along line 6-6 in FIG. 1, with supplies not shown for clarity;
FIG. 7 is a flowchart of an exemplary embodiment of the present invention;
FIG. 8A is a perspective view of an exemplary embodiment of the present invention;
FIG. 8B is an exploded perspective view of an exemplary embodiment of the present invention;
FIG. 8C is a section view of an exemplary embodiment of the present invention, taken along line 8C-8C in FIG. 8A;
FIG. 9A is a perspective view of an exemplary embodiment of the present invention;
FIG. 9B is an exploded perspective view of an exemplary embodiment of the present invention;
FIG. 9C is a section view of an exemplary embodiment of the present invention, taken along line 9C-9C in FIG. 9A;
FIG. 10A is a perspective view of an exemplary embodiment of the present invention;
FIG. 10B is a section view of an exemplary embodiment of the present invention, taken along line 10B-10B in FIG. 10A;
FIG. 11A is a perspective view of an exemplary embodiment of the present invention; and
FIG. 11B is a section view of an exemplary embodiment of the present invention, taken along line 11B-11B in FIG. 11A.

### DETAILED DESCRIPTION OF THE INVENTION

The following detailed description is of the best currently contemplated modes of carrying out exemplary embodiments of the invention. The description is not to be taken in a limiting sense, but is made merely for the purpose of illustrating the general principles of the invention, since the scope of the invention is best defined by the appended claims.

Broadly, an embodiment of the present invention provides a disposable applicator and disposable tray for handling, drying, and separately organizing medical supplies during a medical procedure. The disposable applicator can be used for drying medical supplies. The disposable tray provides two spaced apart recesses for organizing and separating pre-use and post-use medical supplies for preventing cross contamination. The post-use medical supplies, disposable applicator, and the disposable tray can be discarded after the related medical procedure to further prevent the spread of bacteria and viruses.

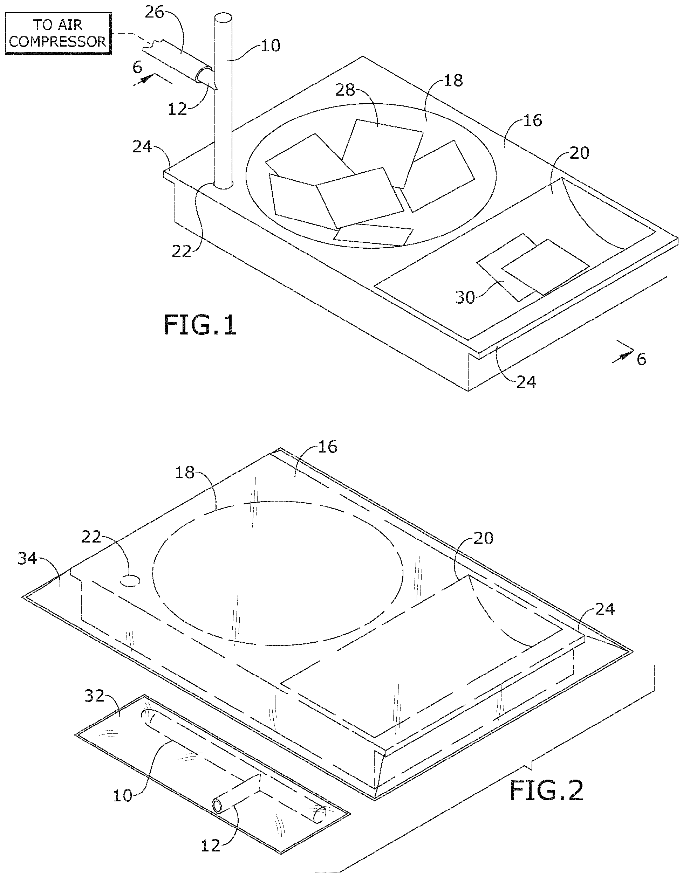

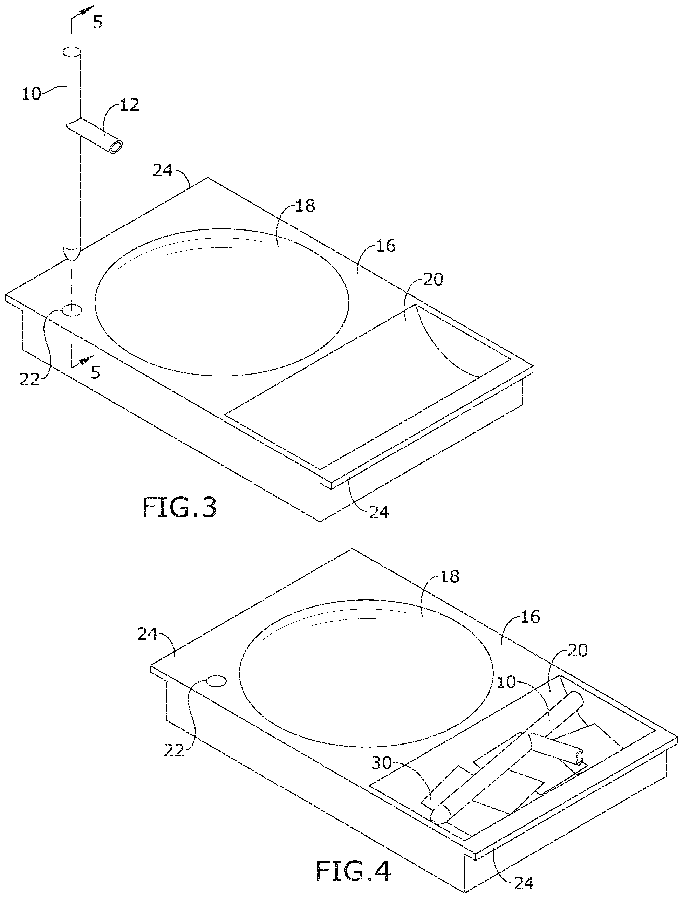

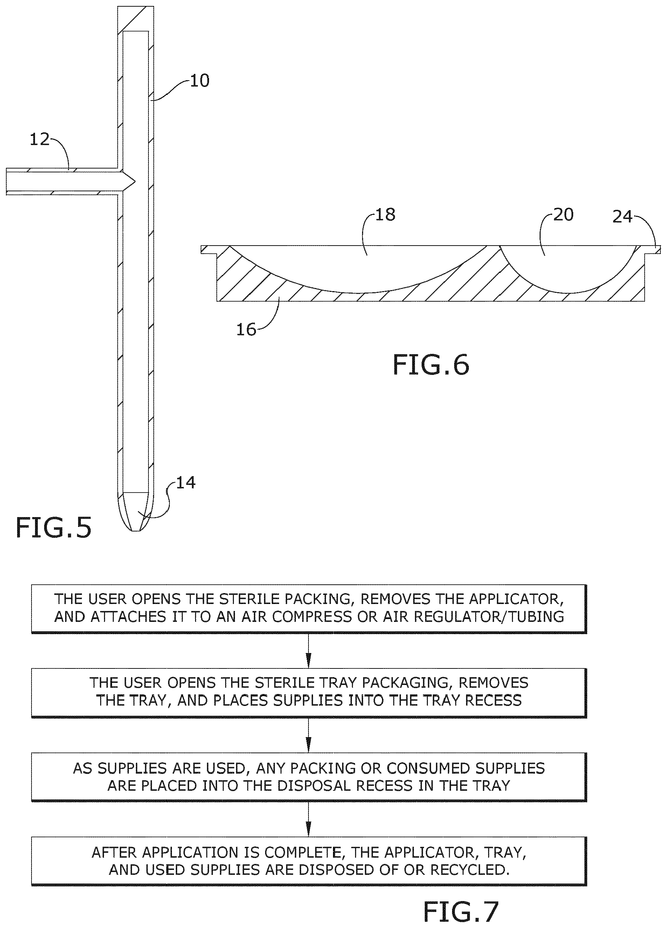

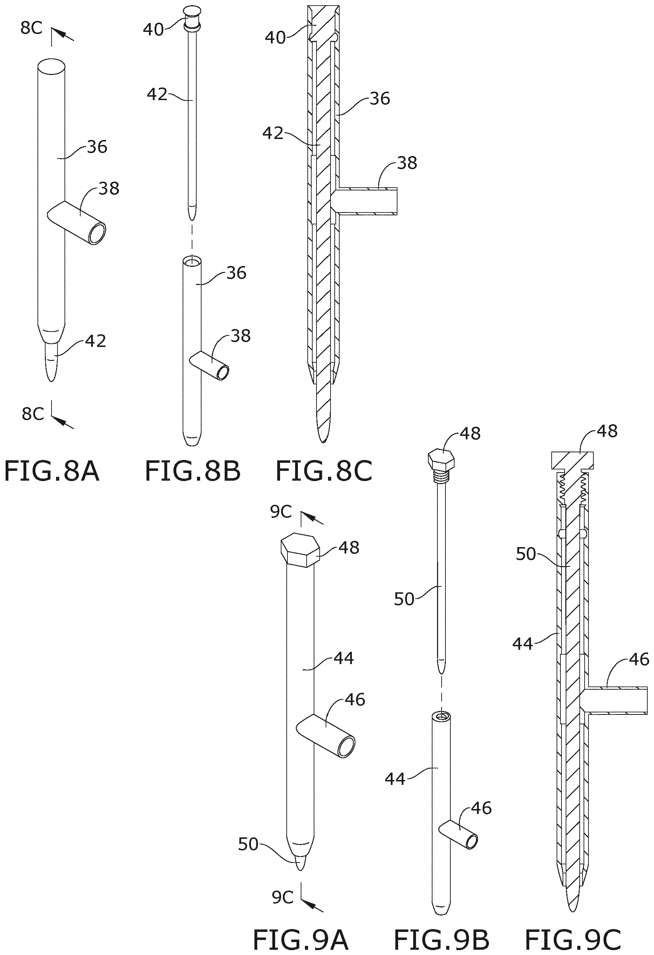

Referring to FIGS. 1 through 11B, the present invention may include a disposable applicator 10 and a disposable tray 16.

The disposable tray 16 may provide a storage retainer 22 for removably securing the disposable applicator 10. The storage retainer 22 may be a hole dimensioned to slidably receive an end or tip 56 of the disposable applicator 10 or 52. The disposable tray 16 may have a first recess 18 and a second recess 20. The disposable tray 16 may have a lip 24 along an upper periphery.

The first recess 18 for supporting pre-use medical supplies 28 and the second recess 20, spaced apart from the first recess 18, wherein the second recess 20 is for supporting post-use ready-for-discard medical supplies 30. It is understood that the first recess 18 may be designated for post-use medical supplies 30 and the second recess 20 for the pre-use medical supplies 28. The pre-use supplies 28 should be opened just before being use on a patient and then discarded into the second recess 20, eliminating the chance of passing infections from one patient to another patient by being reused.

The disposable applicator 10 provides an inlet 12 for fluidly coupling an air supply 26 to an outlet 14 of the disposable applicator 10. The air supply 26 urges (compressed) air through the outlet 14 for, in certain instances, drying the adhesive while applying EEG leads. The disposable applicator 10 may be a housing 36 or 44 dimensioned for slidably receiving a core stem 42 or 50, respectively. Each core stem 42 and 50 extends from a core head 40 and 48, respectively, and a tip that protrudes through the outlet of the housing 36 or 44. In between the core head 40 and 48 and the tip is an inlet 38 and 46, for the housing 36 or 44, respectively, for fluidly coupling to the air supply 26.

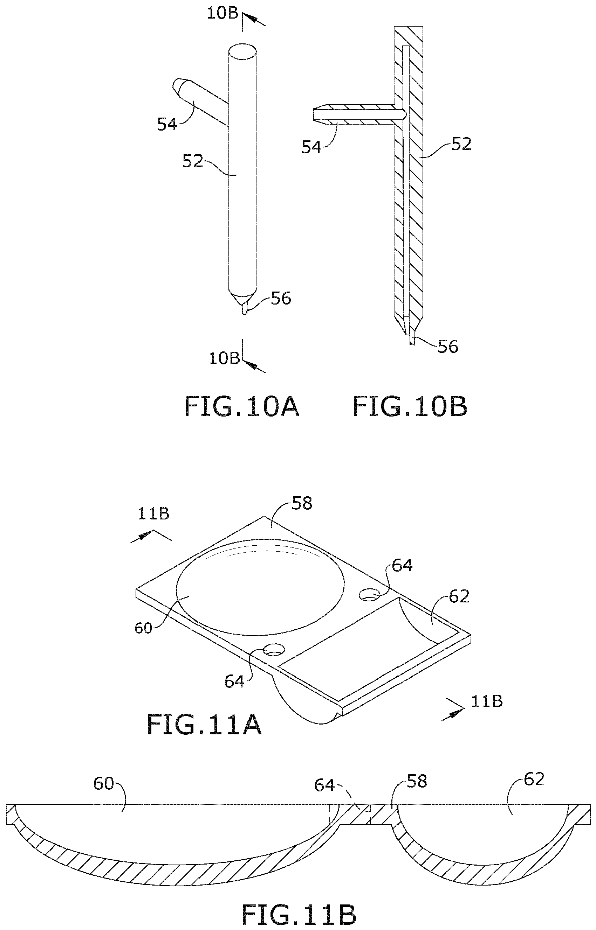

In other embodiments, the applicator 52 may have an inlet 54 as well as an outlet tip 56, wherein ending of 54 is tapered at the end to allow for universal adapters and ease to place on and off, as illustrated in FIGS. 10A and 10B. The outlet tip 56 allows the person to know where the air is going and the outlet tip 56 can be used to poke a hole in gauze to allow, for instance, to allow them to fill the EEG leads later.

In another embodiment, an alternative tray 58 may provide two applicator storage retainers 64 in addition to the first recess 60 and the second recess 62, as illustrated in FIG. 11A and 11B.

Prior to use, for example during shipping and storage, the disposable applicator 10 and disposable tray 16 may be stored in a sealed, sterile container, such as a packaging, 32 and 34, respectively.

The tray 16, 58 and applicator 10, 52 and applicator housing 36 and 44 may be made of any material and can have different shapes as long as they function as disclosed herein.

The tray 16, 58 and applicator 10, 52 and applicator housing 36 and 44 can be used together or independently. A clip (not shown) could be added to the tray 16, 58 to hold the tray 16, 58still if using at bedside.

A method of using the present invention may include the following. The tray 16, 58 and applicator 10, 52 or applicator housing 36 or 44 disclosed above may be provided. One would open the individually wrapped (in the sealed, sterile packaging 32, 34) disposable applicator 10, 52 or disposable applicator housing 36, 44 and attach to an air (compressor or air regulator) supply 12, 38, or 46. Next the individual would open the disposable tray 12, 58 and place the pre-use supplies 28 into the first recess 18 or 60 (or use of the supplies directly from prepackaged container). Once done, the pre-used supplies 28 become post-use supplies 30 and are placed in the second recess 20 or 62. The user may then disposes of or recycles the post-use supplies 30 based on guidelines.

Additionally, the disposable applicator 10, 52 could be used on anything that may need air drying on a small section. The disposable tray 16, 58 could be used for anything to place supplies in while working on a task.

It should be understood, of course, that the foregoing relates to exemplary embodiments of the invention and that modifications may be made without departing from the spirit and scope of the invention as set forth in the following claims.

## Claims

1. A disposable medical supply handling kit, comprising:
a disposable applicator;
a disposable tray having a storage retainer for removably securing the disposable applicator; and
the disposable tray having two spaced apart recesses: a first recess and a second recess.

2. The disposable medical supply handling kit of claim 1, wherein the disposable applicator includes an outlet fluidly coupled to an inlet.

3. The disposable medical supply handling kit of claim 2, further comprising a core stem slidably received in a housing of the disposable applicator, wherein a tip of the core stem protrudes through the outlet.

4. The disposable medical supply handling kit of claim 3, further comprising tip extending from a distal end of the disposable applicator.

5. A disposable tray for managing both pre-use and post-use medical supplies, comprising:
a first recess spaced apart from a second recess.

6. The disposable tray for managing both pre-use and post-use medical supplies of claim 5, further comprising:
one or more storage retainers disposed adjacent the first or second recesses.

7. A method for preventing the contamination of pre-use medical supplies by post-use medical supplies, comprising:
providing the disposable tray of claim 5;
placing pre-use medical supplies in the first recess; and
placing post-use medical supplies in the second recess.
